# EUROPEAN PATENT APPLICATION

(11) **EP 3 127 913 A1**
(43) Date of publication of application: **08.02.2017**
(21) Application number: 15002304.2
(22) Date of filing: 03.08.2015
(51) Int. Cl.: C07K 5/087, C07K 5/09, G01N 33/68

(54) **CHROMOGENIC AND FLUOROGENIC PEPTIDE SUBSTRATES FOR THE DETECTION OF SERINE PROTEASE ACTIVITY**

(71) Applicant: Ruprecht-Karls-Universität Heidelberg, 69117 Heidelberg (DE)
(72) Inventor: Krämer, Roland, 48165 Münster (DE); Arian, Dumitru, 68723 Oftersheim (DE); Harenberg, Job, 69126 Heidelberg (DE)
(74) Representative: Müller-Boré & Partner Patentanwälte PartG mbB

(57) **Abstract**

The present invention relates to chromogenic and fluorogenic substrates that can be used for the highly sensitive and selective detection of the activity of serine proteases. The present invention further relates to methods for the detection of the activity of serine proteases, said methods using the substrates of the present invention. Furthermore, the present invention relates to diagnostic kits and test strips using the above substrates, as well as uses of said substrates.

SEQUENCE LISTING

<110>  Universität Heidelberg

<120>  Chromogenic and Fluorogenic Peptide Substrates For The Detection
       Of Serine Protease Activity

<130>  H 3578EU

<160>  9

<170>  PatentIn version 3.5

<210>  1
<211>  4
<212>  PRT
<213>  Artificial Sequence

<220>
<223>  Serine protease substrate


<220>
<221>  misc
<222>  (1)..(1)
<223>  benzyl-Ile

<220>
<221>  misc
<222>  (2)..(2)
<223>  GluOR (R=H or Me)

<400>  1

Xaa Xaa Gly Arg
1


<210>  2
<211>  4
<212>  PRT
<213>  Artificial Sequence

<220>
<223>  Serine protease substrate


<220>
<221>  misc
<222>  (1)..(1)
<223>  benzyloxycarbonyl-Ile

<220>
<221>  misc
<222>  (2)..(2)
<223>  GluOR (R=H or Me)

<400>  2

Xaa Xaa Gly Arg
1

<210>  3
<211>  4
<212>  PRT
<213>  Artificial Sequence

<220>
<223>  Serine protease substrate


<220>
<221>  misc
<222>  (1)..(1)
<223>  benzoyl-Ile

<400>  3

Xaa Glu Gly Arg
1


<210>  4
<211>  4
<212>  PRT
<213>  Artificial Sequence

<220>
<223>  Serine protease substrate

<400>  4

Ala Ala Val Ala
1


<210>  5
<211>  4
<212>  PRT
<213>  Artificial Sequence

<220>
<223>  Serine protease substrate


<220>
<221>  misc
<222>  (1)..(1)
<223>  succinyl-Ala

<400>  5

Xaa Ala Pro Phe
1


<210>  6
<211>  4
<212>  PRT
<213>  Artificial Sequence

<220>
<223>  Serine protease substrate

<220>
<221>  misc
<222>  (1)..(1)
<223>  benzyl-Ile

<400>  6

Xaa Glu Gly Arg
1


<210>  7
<211>  4
<212>  PRT
<213>  Artificial Sequence

<220>
<223>  Serine protease substrate


<220>
<221>  misc
<222>  (1)..(1)
<223>  tert-butyloxycarbonyl-Leu

<400>  7

Xaa Ser Thr Arg
1


<210>  8
<211>  4
<212>  PRT
<213>  Artificial Sequence

<220>
<223>  Serine protease substrate


<220>
<221>  misc
<222>  (1)..(1)
<223>  methoxysuccinyl-alanine

<400>  8

Xaa Ala Pro Val
1


<210>  9
<211>  4
<212>  PRT
<213>  Artificial sequence

<220>
<223>  Serine protease substrate


<220>
<221>  misc
<222>  (1)..(1)

<223>  glutaryl-alanine

<400>  9

Xaa Ala Pro Leu
1

## Description

The present invention relates to chromogenic and fluorogenic substrates that can be used for the highly sensitive and selective detection of the activity of serine proteases. The present invention further relates to methods for the detection of the activity of serine proteases, said methods using the substrates of the present invention. Furthermore, the present invention relates to diagnostic kits and test strips using the above substrates, as well as uses of said substrates.

There are over 500 proteases, which account for approximately 2% of all proteins in the human body. As one of the largest and most important groups of enzymes, they are involved in many physiological processes, including protein digestion and turnover, blood clotting, apoptosis, hormone activation, fertilization, and growth differentiation. A dysregulated protease activity may be associated with organ dysfunction and consequently with a disease. Therefore, there is a strong demand for the development of sensitive protease assays for proteomic research, disease diagnosis, and drug development and measurement. In addition, triggered protease activation has been applied in cascade-like amplification schemes for the highly sensitive detection of various analytes. The majority of commercial kits for detection of protease activity include a chromogenic or fluorogenic substrate that releases a colored or fluorescent compound upon selective cleavage by the target protease. Among the most widely used chromogenic substrates are peptide *p*-nitroanilides that release yellow colored *p-*nitroaniline with an absorbance maximum at 380 nm and a modest molar absorbance (ε) of 13500 M⁻¹cm⁻¹. Peptide derivatives of 7-amino-4-methylcoumarin (AMC) are widely used fluorogenic substrates that display an increase of 460 nm fluorescence upon release of the fluorophore. These optical properties, however, limit the sensitivity of such substrates for the detection of protease activity in body fluids or tissues due to interference with the colored or autofluorescent biological matrix. Very few protease substrates have been described that liberate colored and/or fluorescent dyes at wavelengths larger than ≈600 nm, corresponding to the edge of the blood and tissue transparency window.

Major protease subgroups are the serine proteases in which serine serves as a nucleophilic amino acid in the active site. Most serine proteases can be classified as trypsin-like proteases that utilize a catalytic triad for activity, composed of highly conserved histidine, aspartate and serine residues. Trypsins, chymotrypsins, and elastases break down polypeptides in the digestive system. Thrombin, factor Xa and other coagulation factors define the blood coagulation and complement cascades. Tryptases are major components in the secretory granules of mast cells, whereas matriptases are membrane bound proteases associated with a variety of cancers. A similar association with cancer is found in the Kallikrein family. Granzymes are mediators of directed apoptosis by natural killer cells and cytotoxic T-cells that play key roles in the defense against viral infection. Because of their abundance and involvement in health and disease, serine proteases and in particular trypsin-like proteases present obvious targets of therapeutic intervention.

This is exemplified by thrombin, the main effector protease of the coagulation cascade, a series of zymogen conversions that is triggered when circulating coagulation factors contact tissue factor. Tissue factor is a type-I integral membrane protein that functions as an obligate cofactor for activation of zymogen factor X by factor VIIa. Factor Xa (with the assistance of cofactor factor Va) then converts prothrombin to active thrombin. Other zymogen conversions provide both amplification and negative feedback loops that regulate thrombin production.

Thrombin is a major therapeutic target for thrombosis and stroke intervention/prevention through indirect inhibitors such as heparin or warfarin, and direct inhibitors such as hirudin (bivalent), and argatroban (monovalent). In addition to its role in thrombosis and stroke, thrombin is reported as a relevant player in cardiovascular disease, renal injury, and cancer.

Concentration of thrombin in blood can vary considerably; indeed, it is not present in blood under normal conditions, but can reach low-micromolar concentrations during the coagulation process. Apart from the haemostatic process, thrombin circulates at the high-picomolar level in the blood of patients suffering from diseases known to be associated with coagulation abnormalities. A variety of methods have been developed to detect thrombin at low concentrations. The majority of these methods requires covalent conjugation of nucleic acid aptamers to nanoparticles (NPs) or electrodes and provides insufficient sensitivity in the low nanomolar range. Recently, it has been shown that gold NPs in conjunction with fibrinogen can detect thrombin in buffer and 10-fold diluted plasma at very low concentrations (0.04 and 0.1 pM, respectively). Other methods determine the thrombin concentration by measuring proteolytic activity using different substrates. The proteolytic activity can be monitored by FRET (Förster resonance energy transfer) through quantum-dot-peptide conjugates, or ratiometric activatable cell-penetrating peptides. The latter substrates provide *in vivo* readout of thrombin activation at the injury site.

A simpler way to measure the enzyme activity is using short peptides conjugated to chromophores or fluorophores as cleavable substrates, wherein *p*-nitroanilides are the most commonly used chromogenic substrates in thrombin assays.

Fluorogenic substrates are of a greater interest as they provide a much more sensitive read-out. Up to now, latent fluorophores (pro-fluorophores) are some of the most widely used tools in visualization of biologically relevant molecules (such as H₂O₂, NO, sulfite, O₃,¹O₂ etc.) and enzyme activities (such as esterases, beta-galactosidase, proteases, ribonucleases). There are only two fluorogenic thrombin substrates currently on the market: 7-amino-4-methylcoumarin (AMC) and rhodamine derivatives.

AMC is widely used to prepare peptidase substrates in which the amide has shorter-wavelength absorption and emission spectra than the amine hydrolysis product (excitation (ex) 360 nm / emission (em) 460 nm; ε = 16.000 M⁻¹cm⁻¹). AMC is partially protonated at low pH (less than ~5) but fully deprotonated at physiological pH. Thus, its fluorescence spectrum is not subject to variability due to pH-dependent protonation/deprotonation when assayed near or above physiological pH. The big disadvantages of this fluorophore are the high emission background in complex biological matrices, low extinction coefficient and low fluorescence quantum yield (ϕ = 0.18). Rhodamine-based substrates provide better results, but because they incorporate two peptide moieties, each serves as a substrate for the enzyme and complicates the interpretation of hydrolysis kinetics.

Chromogenic and fluorogenic substrates for detecting the activity of serine proteases known in the art usually absorb and emit light at lower wavelengths (e.g. below 570 nm), or show only weak absorption and fluorescence at higher wavelengths (e.g. at 570 nm or more). This renders the use of such substrates difficult in samples having a high optical density, and/or a high autofluorescence, such as whole blood, and/or in samples that are in contact with a surface having a high autofluorescence, and/or in samples containing biological structures that are labeled with further chromogenic and/or fluorogenic substances. In particular, an optically dense sample can impair excitation of the substrates and absorb emitted fluorescent light. Further, autofluorescence or fluorescence from further substances can interfere with the fluorescent light to be detected, thus impairing sensitivity of detection. Moreover, substrates known in the art are often characterized by a low molar absorption (ε) and/or low fluorescence quantum yield (ϕ). Further, some substrates are not commercially available and/or are very expensive.

In view of the above, the technical problem underlying the present invention is the provision of chromogenic and fluorogenic substrates for the highly sensitive and selective detection of the activity of serine proteases in a sample, wherein said substrates should be usable in samples having a high optical density and/or a high autofluorescence, and/or in samples that are in contact with a surface having a high autofluorescence, and/or in samples containing biological structures that are labeled with chromogenic and/or fluorogenic substances. Further, said substrates should be characterized by a high molar absorption and high fluorescence quantum yield, in particular at longer wavelengths.

The solution to the above technical problem is achieved by the embodiments characterized in the claims.

In particular, in a first aspect, the present invention relates to a compound having Formula (I): 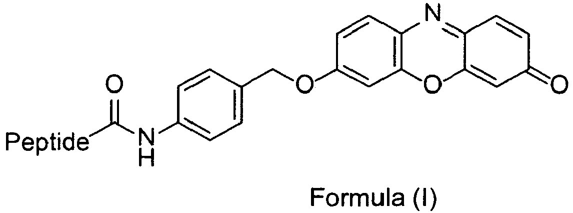 wherein
Peptide is a peptide or peptide derivative, or a salt of said peptide or peptide derivative.

In a preferred embodiment, Peptide is a peptide or peptide derivative, preferably a tripeptide or tripeptide derivative, whose C-terminal amino acid is arginine or lysine, preferably arginine. The salt of the peptide or peptide derivative according to the present invention is preferably a chloride, acetate, or trifluoroacetate salt.

In preferred embodiments, the peptide or peptide derivative is selected from the group consisting of thrombin substrates, factor Xa substrates, trypsin substrates, chymotrypsin substrates, factor VIIa substrates, factor IXa substrates, factor XIa substrates, factor XIIa substrates, kallikrein substrates, plasmin substrates, tissue plasminogen activator substrates, activated protein C substrates, tryptase substrates, matriptase substrates, granzyme substrates, elastase substrates, and human complement protease C1r substrates.

In this context, preferred thrombin substrates are D-Phe-Pro-Arg, D-Phe-HomoPro-Arg, Tos-Gly-Pro-Arg, Boc-Val-Arg, Boc-Val-Pro-Arg, Boc-Asp(O-Benzyl)-Pro-Arg, Bz-Phe-Val-Arg, Sar-Pro-Arg, Z-Gly-Gly-Arg, Z-Pro-Arg, Ethylmalonate-Gly-Arg, beta-Ala-Gly-Arg, Moc-Gly-Pro-Arg, D-CHG-Ala-Arg, D-CHG-Pro-Arg, D-CHG-But-Arg, D-CHA-Gly-Arg, and D-CHA-Ala-Arg
(wherein: HomoPro is homoproline, Tos is p-toluenesulfonyl, Boc is *tert-*butyloxycarbonyl, Bz is benzyl, Z is Benzyloxycarbonyl, Moc is methoxycarbonyl, CHG is cyclohexylglycine, CHA is 3-cyclohexylalanine, Sar is sarcosine, and But is 2-aminobutyric acid).

Further, preferred factor Xa substrates are D-Arg-Gly-Arg, Bz-Ile-GluOR-Gly-Arg (R=H, Me; SEQ ID NO: 1), Z-Ile-GluOR-Gly-Arg (R=H, Me; SEQ ID NO: 2), Suc-Ile-Glu(gammaPip)-Gly-Arg, Z-D-Arg-Gly-Arg, Boc-D-Arg-Gly-Arg, Ets-D-Arg-Gly-Arg, Bs-D-Arg-Gly-Arg, 4-Nz-D-Arg-Gly-Arg, 4-Nbs-D-Arg-Gly-Arg, Tos-D-Arg-Gly-Arg, Moz-D-Arg-Gly-Arg, Mbs-D-Arg-Gly-Arg, 4-CIBs-D-Arg-Gly-Arg, Ns-D-Arg-Gly-Arg, Bzls-D-Arg-Gly-Arg, Eoc-D-Arg-Gly-Arg, Mes-D-Arg-Gly-Arg, Z-D-Arg-Sar-Arg, Ac-D-Arg-Gly-Arg, Moc-D-CHA-Gly-Arg, Moc-D-CHG-Gly-Arg, Moc-D-Val-Gly-Arg, Mes-D-CHA-Gly-Arg, Moc-D-Nle-Gly-Arg, and Mes-D-Leu-Gly-Arg
(wherein Bz is benzyl, Z is benzyloxycarbonyl, Suc is succinyl, Pip is piperazine, Boc is *tert*-butyloxycarbonyl, Ets is ethanesulfonyl, Bs is benzenesulfonyl, 4-Nz is 4-nitrobenzyloxycarbonyl, 4-Nbs is 4-nitrobenzenesulfonyl, Tos is p-toluenesulfonyl, Moz is 4-methoxybenzyloxycarbonyl, Mbs is 4-methoxybenzenesulfonyl, 4-CIBs is 4-chlorobenzenesulfonyl, Ns is beta-naphthalenesulfonyl, Bzls is benzylsulfonyl, Eoc is ethyloxycarbonyl, Mes is methanesulfonyl, Sar is sarcosine, Ac is acetyl, Moc is methoxycarbonyl, CHA is cyclohexylalanine, CHG is cyclohexylglycine, and Nle is norleucine).

Further, preferred trypsin substrates are Bz-Ile-Glu-Gly-Arg (SEQ ID NO: 3), Bz-Phe-Val-Arg, Boc-Gln-Ala-Arg, Bz-Val-Gly-Arg, Boc-Val-Pro-Arg, Boc-Glu(O-Benzyl)-Gly-Arg, Z-Gly-Gly-Arg, Z-Phe-Val-Arg, Boc-Gln-Gly-Arg, Z-Val-Gly-Arg, and Z-D-Ala-Gly-Arg
(wherein Bz is benzyl, Boc is *tert*-butyloxycarbonyl, Bz is benzyl, and Z is benzyloxycarbonyl).

Further, preferred chymotrypsin substrates are Ala-Ala-Phe, Ala-Ala-Val-Ala (SEQ ID NO: 4), Glutaryl-Ala-Ala-Phe, Suc-Ala-Ala-Pro-Phe (SEQ ID NO: 5), and Suc-Gly-Gly-Phe
(wherein Suc is succinyl).

Further, preferred factor VIIa substrates are Bz-Ile-Glu-Gly-Arg (SEQ ID NO: 6), Boc-Leu-Thr-Arg, and Mes-D-CHA-Abu-Arg
(wherein Bz is benzyl, Boc is *tert*-butyloxycarbonyl, Mes is methanesulfonyl, CHA is cyclohexylalanine, and Abu is aminobutyric acid).

Further, preferred factor IXa substrates are Mes-D-CHG-Gly-Arg, and D-Leu-PHG-Arg
(wherein Mes is methanesulfonyl, CHG is cyclohexylglycine, and PHG is phenylglycine).

Further, preferred factor XIa substrates are pyroGlu-Pro-Arg, and Z-Aad-Pro-Arg (wherein pyroGlu is pyroglutamic acid, Z is benzyloxycarbonyl, and Aad is alpha-aminoadipic acid).

Further, preferred factor Xlla substrates are Boc-Gln-Gly-Arg, Bz-Ile-Glu-Gly-Arg (SEQ ID NO: 6), and D-CHA-Gly-Arg
(wherein Boc is *tert*-butyloxycarbonyl, Bz is benzyl, and CHA is cyclohexylalanine).

Further, preferred kallikrein substrates are Pro-Phe-Arg, D-Pro-Phe-Arg, Val-Leu-Arg, D-Val-Leu-Arg, Bz-Pro-Phe-Arg, D-Val-CHA-Arg, and D-Abu-CHA-Arg (wherein Bz is benzyl, CHA is cyclohexylalanine, and Abu is alpha-aminobutyric acid).

Further, preferred plasmin substrates are Gly-Arg, D-Val-Leu-Lys, D-Val-Phe-Lys, pyroGlu-Phe-Lys, Tos-Gly-Pro-Lys, D-Ile-Phe-Lys, Suc-Ala-Phe-Lys, Isovaleryl-Phe-Lys, Boc-Val-Leu-Lys, Boc-Glu-Lys-Lys, Ala-Phe-Lys, D-Ala-Leu-Lys, D-Ala-Phe-Lys, Z-Ala-Ala-Lys, D-Ala-CHA-Lys, D-But-CHA-Lys, D-Nva-CHA-Lys, and D-Nle-CHA-Lys
(wherein pyroGlu is pyroglutamic acid, Tos is *p*-toluenesulfonyl, Suc is succinyl, Boc is *tert*-butyloxycarbonyl, Z is benzyloxycarbonyl, CHA is cyclohexylalanine, Nva is norvaline, and Nle is norleucine).

Further, preferred tissue plasminogen activator substrates are D-Ile-Pro-Arg, D-Val-Gly-Arg, Z-Gly-Gly-Arg, Gly-Gly-Arg, Glutaryl-Gly-Arg, D-Val-Leu-Lys, Mes-D-CHA-Gly-Arg, Mes-D-Phe-Gly-Arg, and Mes-D-Abu-Gly-Arg
(wherein Z is benzyloxycarbonyl, Mes is methanesulfonyl, and Abu is aminobutyric acid).

Further, preferred activated protein C substrates are pyroGlu-Pro-Arg, Boc-Leu-Ser-Thr-Arg (SEQ ID NO: 7), D-Lys(Z)-Pro-Arg, D-CHA-Pro-Arg, and pyroGlu-CHG-Arg
(wherein pyroGlu is pyroglutamic acid, Boc is *tert*-butyloxycarbonyl, Z is benzyloxycarbonyl, CHA is cyclohexylalanine, and CHG is cyclohexylglycine.)

Further, preferred tryptase substrates are pyroGlu-Pro-Arg, Boc-Phe-Ser-Arg, Boc-Val-Pro-Arg, D-Leu-Thr-Arg, Tos-Gly-Pro-Lys, and Ac-Lys-Pro-Arg (wherein pyroGlu is pyroglutamic acid, Boc is *tert*-butyloxycarbonyl, Tos is p-toluenesulfonyl, and Ac is acetyl).

Further, preferred elastase substrates are Succinyl-Ala-Ala-Ala, Methoxysuccinyl-Ala-Ala-Pro-Val (SEQ ID NO: 8), Succinyl-Ala-Pro-Ala, pyroGlu-Pro-Val, and Glutaryl-Ala-Ala-Pro-Leu (SEQ ID NO: 9)
(wherein pyroGlu is pyroglutamic acid).

Furthermore, a preferred complement protease C1r substrate is Z-Gly-Arg (wherein Z is benzyloxycarbonyl).

Moreover, preferred matriptase substrates are Boc-Gln-Ala-Arg, and Z-Gly-Pro-Arg
(wherein Boc is *tert*-butyloxycarbonyl, and Z is benzyloxycarbonyl).

Finally, a preferred granzyme substrate is Z-Gly-Pro-Arg (wherein Z is benzyloxycarbonyl).

In a particularly preferred embodiment, Peptide is the peptide D-Phe-Pro-Arg (Compound **1**) or a salt thereof. In an equally preferred embodiment, Peptide is the peptide derivative Benzylsulfonyl-D-Arg-Gly-Arg (Compound **2**) or a salt thereof.

In this context, the term "D-Xaa", wherein Xaa is any amino acid, denotes the respective D-amino acid. All other amino acids are L-amino acids. Further, the rightmost amino acid in all of the above sequences is the amino acid that is bound to the amino group of the compounds of the present invention to which the group "Peptide" is bound.

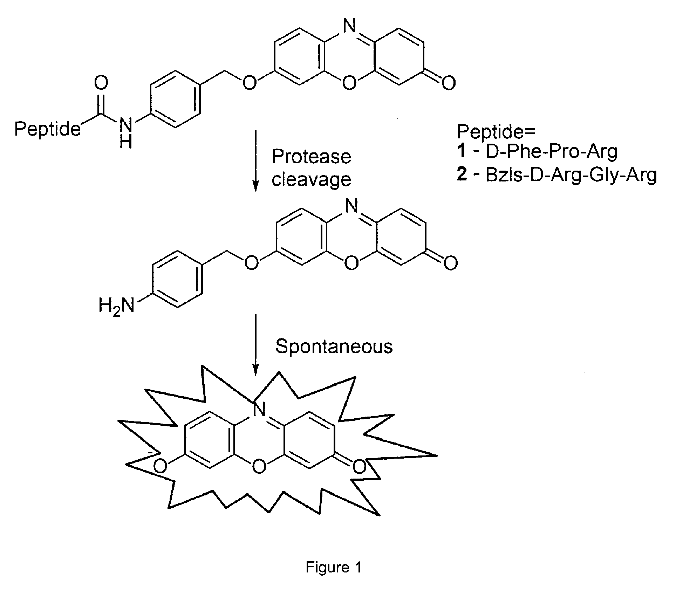

Upon cleavage of the compounds of the present invention by the serine protease to be detected, an intermediate compound is formed which spontaneously converts to the chromogenic and fluorogenic compound resorufin (7-Hydroxy-3H-phenoxazin-3-one) (Fig. 1).

Methods and means for synthesizing the compounds of the present invention are not particularly limited and are known in the art. Preferably, synthesis is achieved as indicated in the Examples of the present application.

In a second aspect, the present invention relates to a method for the detection of the activity of at least one serine protease in a sample, comprising the steps of contacting said sample with a compound according to the present invention, and measuring the amount of resorufin released from said compound.

As used herein the term "detection of the activity of at least one serine protease" encompasses the qualitative and/or quantitative detection/determination of the activity of said protease. In particular, qualitative detection determines if activity is present or not, and quantitative detection determines protease activity in *enzyme units (U* or *units*), defined as the amount of protease that converts one µmole substrate per minute under standard conditions, or in *katal* (*kat*), defined as the amount of protease that converts one mole of substrate per second under standard conditions. Methods for determining presence or absence of protease activity, as well as for quantitatively determining protease activity are not particularly limited and are known in the art. Quantitative determination of protease activity can for example be achieved by establishing a standard curve using samples of known protease activity and relating the samples of interest to said standard curve.

Serine proteases that can be analyzed using the methods of the present invention are only limited by their specificity for the peptide portion of the compounds of the present invention and are known in the art. In a preferred embodiment, the serine protease is a trypsin-like protease, preferably a trypsin-like protease selected from the group consisting of trypsins, chymotrypsins, elastases, thrombin, factor VIIa, factor IXa, factor Xa, factor Xla, factor Xlla, kallikreins, plasmin, tissue plasminogen activator, activated protein C, human complement protease C1 r, tryptases, matriptases, and granzymes. In this context, thrombin and factor Xa are each particularly preferred.

Substrates for use in the detection of the above proteases are preferably selected from the group consisting of the substrates indicated above.

In a particularly preferred embodiment, the trypsin-like protease whose activity is to be detected is thrombin, and the compound used in the method of the present invention is Compound **1** or a salt thereof. In an equally preferred embodiment, the trypsin-like protease whose activity is to be detected is factor Xa, and the compound used in the method of the present invention is Compound **2** or a salt thereof.

Samples in which the activity of serine proteases can be determined according to the methods of the present invention are not particularly limited. However, the particular advantages of said methods are best employed in samples having a high optical density, in particular a high optical density at wavelengths of below 570 nm, and/or a high autofluorescence, in particular a high autofluorescence at wavelengths of below 570 nm.

In another embodiment, the sample is in contact with a surface having a high autofluorescence. This surface can be part of *e.g*. a microtiter plate, a glass or plastic cuvette, or a microscope slide or cover slip, which all can show high amounts of autofluorescence at certain wavelengths which can substantially impair excitation and/or emission of chromogenic and/or fluorogenic substances. In a further embodiment, the sample contains at least one biological structure that is labeled with at least one chromogenic and/or fluorogenic substance. This embodiment relates e.g. to samples in which certain markers of interest have been labeled with e.g. antibodies having a conjugated fluorochrome, which can also impair excitation and/or emission of chromogenic and/or fluorogenic substances used for detecting the activity of serine proteases.

Particular examples of samples/sample materials include whole blood, serum, plasma, urine, saliva, sputum, semen, lacrimal fluid, cerebrospinal fluid, defecation, cells and tissues, wherein whole blood, plasma, serum and urine are particularly preferred.

In certain embodiments, the methods of the present invention can be used for the detection of inhibitors of serine proteases. In these embodiments, the sample is spiked with a respective serine protease and the activity thereof detected.

The step of contacting the sample with a compound of the present invention according to the methods of the present invention is preferably performed at conditions in which the serine proteases can exert their function, *i.e*. in which protease activity is possible, and in which the compounds of the present invention are stable. Respective conditions are known to a person skilled in the art.

The step of measuring the amount of resorufin released from the compounds of the present invention according to the methods of the present invention is not particularly limited and encompasses methods known in the art. Such methods include for example colorimetric and/or fluorimetric methods known in the art.

In a third aspect, the present invention relates to a test strip having a surface on which a compound of the present invention is immobilized. According to a particular embodiment, a protease may be immobilized on the surface of the test strip in addition to the compound of the present invention. According to this embodiment, the test strip of the present invention may be used for the detection of a protease inhibitor.

In this aspect, all definitions and limitations defined for the compounds of the present invention according to the first aspect of the invention apply in an analogous manner.

Respective test strips are not particularly limited and are known in the art.

Means for immobilizing the compounds of the present invention on the test strips are not particularly limited and are known in the art.

In a fourth aspect, the present invention relates to the compounds of the present invention according to the first aspect of the invention for use in the diagnosis of a condition or disease in a subject that is characterized by abnormal levels of at least one serine protease.

In this aspect, all definitions and limitations defined for the compounds of the present invention according to the first aspect of the invention, and the methods of the present invention according to the second aspect of the present invention, apply in an analogous manner.

Conditions or diseases that are characterized by abnormal levels of at least one serine protease are preferably selected from the group consisting of postoperative period with overwhelming thrombin formation and/or enhanced risk of venous thrombosis, pulmonary embolism, pulmonary fibrosis and cancers, arterial thromboembolism, cardiovascular disease, renal injury, and impaired thrombin formation predisposing patients to enhanced bleeding. These examples document that plasma levels of free thrombin represent a promising biomarker reflecting a patient's individual hemostatic status to guide successful treatment decisions.

In a preferred embodiment, the subject is a human subject.

The term "abnormal levels of at least one serine protease" as used herein relates to levels of a respective protease that are higher or lower as compared to healthy control subjects. While normal blood levels of active thrombin are very low and difficult to detected by currently available assays, peak concentration during hip surgery may exceed 100 pM.

In a related fifth aspect, the present invention relates to a method of diagnosing a condition or disease in a subject that is characterized by abnormal levels of at least one serine protease, comprising the steps of providing a sample from the subject, contacting said sample with a compound according to the present invention, and measuring the amount of resorufin (7-Hydroxy-3H-phenoxazin-3-one) released from said compound.

In this aspect, all definitions and limitations defined for the compounds of the present invention according to the first aspect of the invention, the methods of the present invention according to the second aspect of the present invention, and the compounds for use according to the fourth aspect of the present invention, apply in an analogous manner.

Preferably, the method of diagnosing according to the present invention is an *in vitro* method. Further, the step of providing a sample from the subject is preferably expressly intended to exclude the actual obtaining of said sample.

In a sixth aspect, the present invention relates to uses of the compounds of the present invention for the detection of the activity of at least one serine protease in a sample.

In this aspect, all definitions and limitations defined for the compounds of the present invention according to the first aspect of the invention, and the methods of the present invention according to the second aspect of the present invention, apply in an analogous manner.

In a final seventh aspect, the present invention relates to a diagnostic kit, said kit comprising at least one compound of the present invention. Preferably, said kit further comprises means for performing the methods of the present invention according to the above second and/or fifth aspect. In another embodiment, said kit comprises at least one test strip of the present invention. In this context, means for performing the methods of the present invention according to the above second and/or fifth aspect are not particularly limited and are known in the art.

In preferred embodiments, in case of a diagnostic kit for assaying the concentration or presence of a protease, said kit may comprise a reaction medium such as a buffer solution or lyophilized buffer, and a calibrator or standard containing the protease, in addition to the at least one compound of the present invention. In other preferred embodiments, in case of a diagnostic kit for assaying the concentration or presence of a protease inhibitor, said kit may comprise a reaction medium such as a buffer solution or lyophilized buffer, the protease itself, and a calibrator or standard containing the inhibitor, in addition to the at least one compounds of the present invention.

In this aspect, all definitions and limitations defined for the compounds of the present invention according to the first aspect of the invention, and the methods of the present invention according to the second and fifth aspect of the present invention, apply in an analogous manner.

As used herein, the terms "comprising/comprises", "consisting of/consists of" and "consisting essentially of/consists essentially of" are used interchangeably, *i.e.*, each of said terms can expressly be exchanged against one of the other two terms.

The present invention provides novel chromogenic protease substrates based on resorufin, a highly colored and highly fluorescent, red-emissive dye. Application of the substrates is exemplified by the highly sensitive detection of the trypsin-like coagulation proteases thrombin and factor Xa as well as of the thrombin inhibitor dabigatran in human plasma and whole blood. Point-of-care testing of new oral anticoagulants, including the blockbuster drugs dabigatran (marketed as Pradaxa®) and rivaroxaban (marketed as Xarelto®), is an unmet need.

Resorufin-based substrates provide significantly greater sensitivity in fluorescence-based assays due to lower background absorbance and fluorescence. It is a longer wavelength dye (ex 570 nm / em 585 nm) with higher quantum yield (ϕ = 0.75) and extinction coefficient (ε = 60.000 M⁻¹cm⁻¹). Further, the relatively low pKa of resorufin (-6.0) permits continuous measurement of enzymatic activity.

Described herein is the synthesis, characterization and preliminary studies of a fluorogenic and chromogenic probe for detection of serine proteases such as thrombin and factor Xa with high selectivity and sensitivity. It is also shown that dabigatran, a commonly used anticoagulant, can be detected in plasma as well as in whole-blood, by using the compounds of the present invention, which makes it very attractive for diagnostics. The present invention may also have applications in whole-blood thrombin generation assays.

The figures show:
Figure 1:
   Activation of pro-fluorophore by proteolytic enzymes
   After cleavage of the compounds of the present invention by a protease, an intermediate compound is formed which spontaneously converts to the chromogenic and fluorogenic compound resorufin.
Figure 2:
   Synthesis of Compound **1** of the present invention
   Reagents and conditions: (a) TBTU, DIEA, DMF, rt, 12h (93%); (b) cyanuric chloride, DMSO, rt, 1 h (30%); (c) K₂CO₃, DMF, rt, 12h (95%); (d) TFA-DCM 1:1, rt, 3h (88%); (e) TBTU, DIEA, DMF, rt, 12h (73%); (f) THF-H₂O, NaOH, 0°C, 3h (79%); (g) TBTU, DIEA, DMF, rt, 12h; (h) TFA-DCM 1:1, rt, 1 h (60% over 2 steps).
Figure 3:
   Synthesis of Compound **2** of the present invention
   Reagents and conditions: (a) NaOH, Bzls-Cl, Et₃N, acetone-water (27%); (b) NHS-ester: NHS, DCC, DME then NaHCO₃ (19%); (c) TBTU, DIEA, DMF, rt, 12h; (d) TFA-DCM 1:1, rt, 3h (10% over 2 steps).
Figure 4:
   A. Absorption spectra of resorufin and compound **1** of the present invention;
   B. Enzymatic hydrolysis of **1** (5 µM) in the presence of thrombin (100pM) with Tris buffer pH 8.3 (λₑₓ = 570 nm; λₑₘ = 583 nm) at 24°C; inset: full emission spectra recorded after 60 min, with and without enzyme; C. Emission spectra demonstrating the stability of **1** in Tris buffer pH 8.3, at 24°C.
Figure 5:
   Kinetic parameters obtained for compound **1** (A) and compound **2** (B).
Figure 6:
   Fluorescence assay to evaluate the specificity of compound **1** toward thrombin
   The enzymatic reactions were carried out in 50 mM Tris buffer pH 8.3 and 130 mM NaCl. The activity shows the increase of resorufin fluorescence over time.
Figure 7:
   Representative standard curve for determination of thrombin in water.
   The mean change of resorufin fluorescence dF/min is plotted versus the corresponding thrombin concentration (in pM).
Figure 8:
   Calibration curve for determination of dabigatran concentration in human plasma
   The measurements were carried out in triplicate. Plasma spiked with dabigatran was added to a thrombin solution and assayed at the 1:25 dilution. The thrombin solution had the following composition: human thrombin 100 pM; Tris buffer pH 8.3 50 mM; NaCl 130 mM; urea 500 mM; BSA 0.01%; polybrene 100 ng/mL; aprotinin 0.15 U/mL. Compound **1** of the present invention (5 µM) was added after 5 min preincubation of plasma sample with thrombin solution. The inverted reaction rate of the enzyme with the substrate, determined from the increase of resorufin fluorescence in time, was plotted versus the dabigatran concentration. The picture shows the reaction wells with different dabigatran concentrations after 60min (taken under UV-lamp).
Figure 9:
   Calibration curve for determination of dabiqatran concentration in whole human blood
   The fresh blood sample (20 µL) was stabilized with 20 mM EDTA solution (2 µL), which also contained dabigatran at desired concentration. The thrombin solution had the following composition: human thrombin 250 pM; Tris buffer pH 8.3 50 mM; NaCl 130 mM; urea 500 mM; BSA 0.01%; polybrene 100 ng/mL; aprotinin 40 mU/mL. The fluorogenic substrate 1 (10 µM) was added after 5 min preincubation of blood sample with thrombin solution. The reaction rate of the enzyme with the substrate, determined from the increase of resorufin fluorescence in time, is plotted versus the dabigatran concentration.

The present invention will be further illustrated by the following examples without being limited thereto.

### Examples

### Example 1:

### Probe Design

Successful probes for biomolecular imaging applications need to fulfill several requirements: increase in emission intensity upon reaction with the enzyme, efficiency and stability. Herein, the model of a self-cleavable linker as spacer between peptide substrate and the fluorescent label was chosen. The prodrug linker *p*-aminobenzyl alcohol (PABA) allows coupling of peptides through its amino group and conjugation of alcohol and aniline-based fluorophores and drugs. The spacer is also beneficial to prevent steric hindrance around the cleavage site. As the fluorescent reporter molecule, resorufin (Res) was chosen which can be used to visualize enzyme activities. Very important features are good solubility in water, long emission wavelength and extremely efficient quenching via 7-hydroxy substitution.

To evaluate the efficiency of thrombin and factor Xa to activate the probes bearing the PABA spacer, D-Phe-Pro-Arg-PABA-Res (1) (thrombin substrate) and Bzls-D-Arg-Gly-Arg-PABA-Res (2) (factor Xa substrate) were synthesized (Figure 1).

### Example 2:

### Synthesis

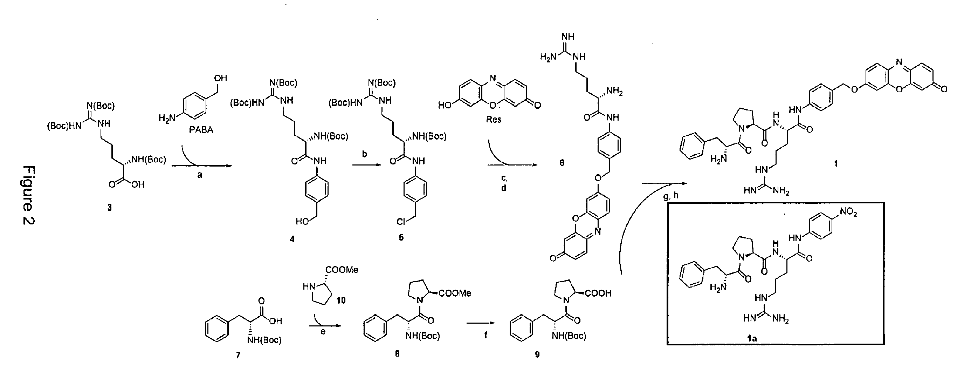

The synthesis of the building block **6**, which was used for both enzyme substrates started from Boc-Arg(Boc)₂-OH (**3**) (Figure 2). It coupled to PABA under standard conditions affording the benzylic alcohol (**4**) in almost quantitative yield. The chlorination of the alcohol proved to be very tricky due to acid-labile Boc groups. Commonly used chlorination reagents (SOCl₂/Et₃N, CCl₄/Ph₃P) failed to deliver the desired compound. Even MsCl/Et₃N, a mild chlorination reagent, afforded **5** in unsatisfactory yields (9-15%). The best results were obtained with a cyanuric chloride/DMSO mixture, which gave **5** in 30% yield. It is worthwhile mentioning that 0.5eq of cyanuric chloride was optimal; more reagent led to dramatic decrease of isolated product. Alkylation of resorufin and Boc-deprotection proceeded smoothly yielding the conjugate **6** in very good yield.

As shown in the Figure 2, the synthesis of the second building block started with the coupling of Boc-D-Phe-OH (7) with H-Pro-OMe (**10**) to provide the methyl ester **8** in good yield. The ester was hydrolyzed in THF/H₂O mixture with NaOH at 0°C, providing the free dipeptide in 79% yield.

Coupling of the dipeptide **9** with the building block **6** was best achieved using TBTU as activator in the coupling reaction. COMU was also used, but some byproducts which form are difficult to separate from the product. The conjugate was chromatographed on a column packed with C18 silica. The final Boc-deprotection with TFA/DCM mixture afforded **1** in good yield as TFA salt.

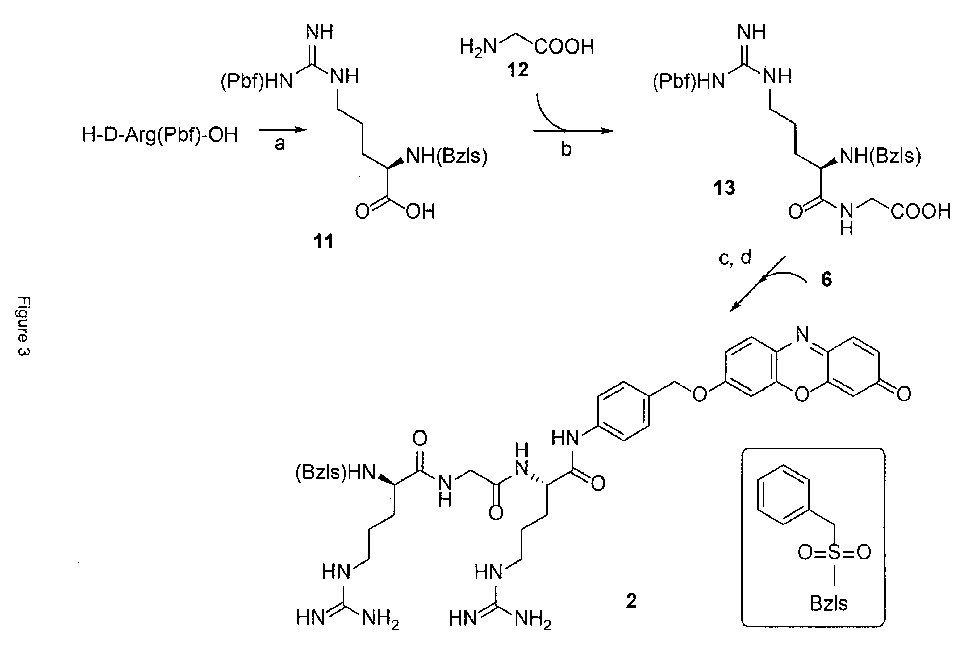

The Factor Xa substrate **2** was synthesized similarly (Figure 3). H-D-Arg(Pbf)-OH was first protected with benzylsulfonyl chloride. The coupling with glycine via NHS-ester afforded dipeptide **13**, which was further coupled to the building block **6** following the same procedure as used for the preparation of **1**.

### Example 3:

### Substrate Properties

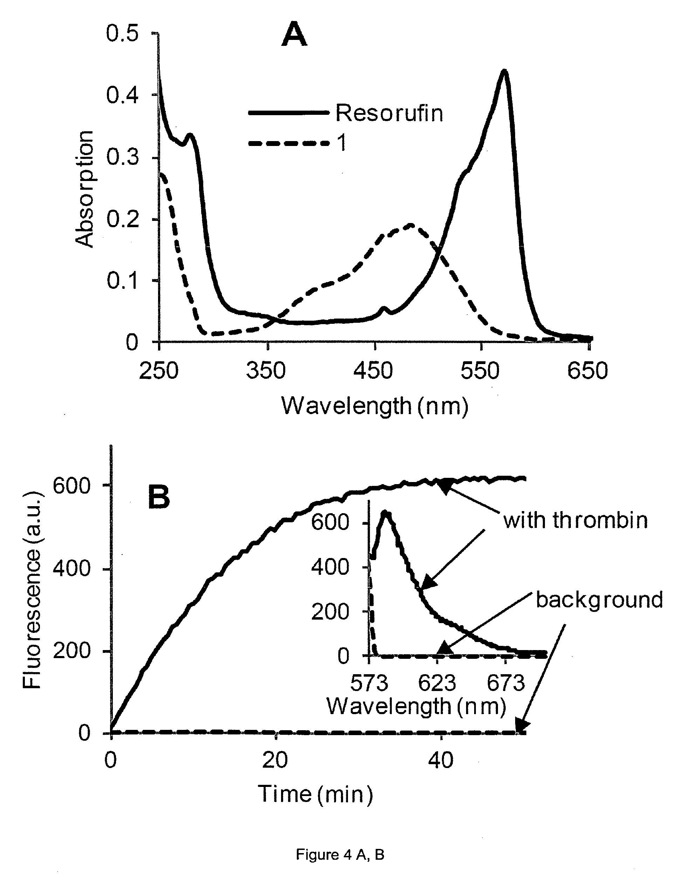

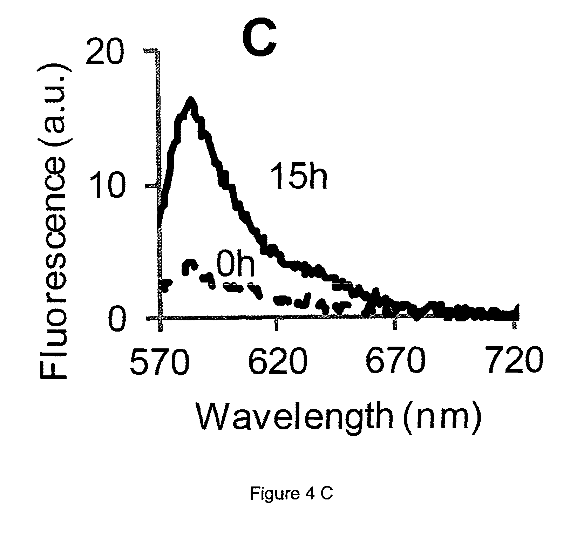

The photophysical properties of compound **1** of the present invention, as well as its enzymatic conversion to fluorescent product resorufin were investigated. Compound **1** displays a blue shift in the absorption spectra (-90 nm) relative to resorufin (Figure 4A). It also has a negligible emission, if exited either at its maxima (480 nm) or at resorufin maxima (570 nm). Additionally, no spontaneous hydrolysis is observed during incubation with thrombin buffer (Tris pH 8.3), indicating high stability of the conjugate (Figure 4C). Thrombin-induced substrate hydrolysis gives rise to a ~300-fold increase in fluorescence, which demonstrates that quenching of resorufin upon alkylation is extremely efficient (Figure 4B).

### Example 4:

### Kinetic measurements

Commercial substrate **1a**, depicted in Figure 2, was used as reference in the kinetic measurements. The kinetic values *K*_{M}, *k*_{cat} and *k*_{cad}/*K*_{M} were established for the fluorogenic compound **1** using Human Thrombin. In order to compare the results of **1** and **1a**, the absorption of the released chromophores was measured during the enzymatic reaction (570 nm for resorufin and 380 nm for *p*-nitroaniline). Advantageously, thrombin turnover of **1** did not suffer at all, suggesting that the PABA linker plays a very important role in the recognition of the substrate by the enzyme, placing the fluorophore away from the active site.

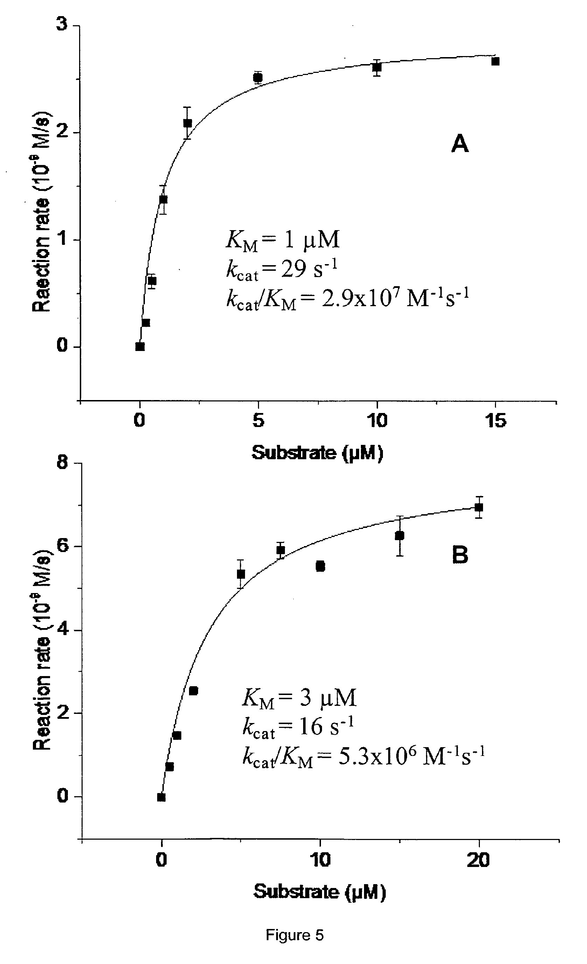

The same parameters were also determined for **1** by measuring the emission of resorufin at 583 nm and similar values to the ones obtained from absorption were found (Figure 5A).

The Factor Xa substrate **2** surprisingly showed a very low *K*_{M} value, a relatively good turnover number *k*_{cat} and excellent catalytic efficiency (Figure 5B). A similar conjugate, with the same peptide sequence, but having *p*-nitroanilide as reporter, has a much higher *K*_{M} (40 µM) and the enzyme has poorer catalytic efficiency for this substrate (2.7×10⁶ M⁻¹ s⁻¹). Thus, compound **2** of the present invention performs better than the best factor Xa substrate so far reported.

### Example 5:

### Selectivity of Fluorogenic Compound 1 and LOP for Thrombin

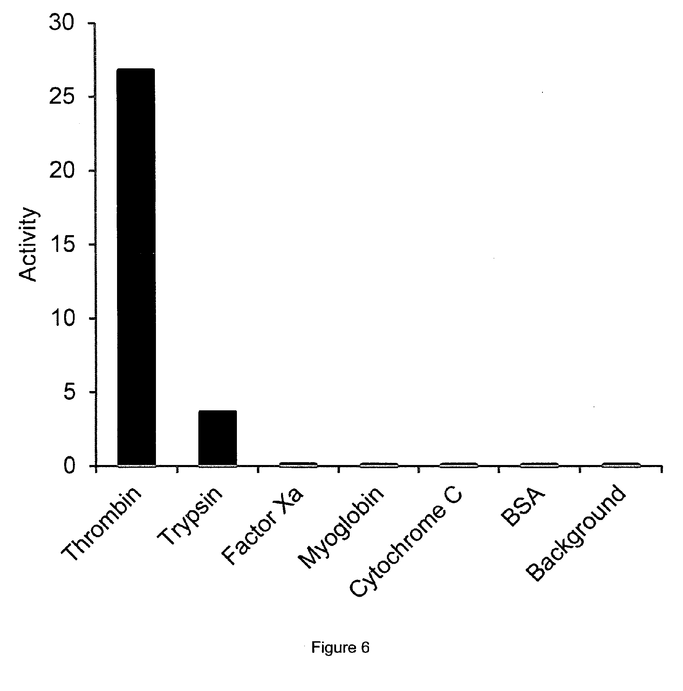

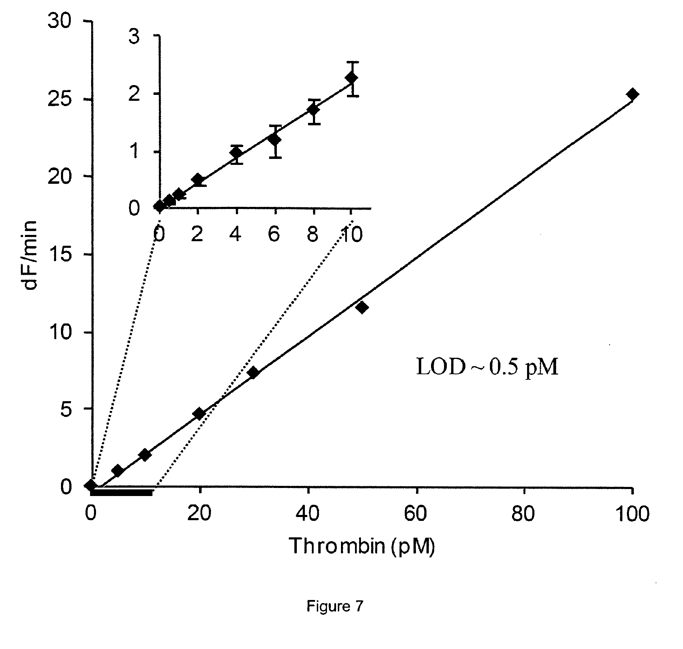

the specificity of Compound **1** of the present invention toward thrombin was tested. Compound **1** (5 µM) was incubated in the presence of thrombin (100 pM) and some possible interfering proteases and proteins, like trypsin, factor Xa, myoglobin, cytochrome C and BSA (100 pM). The increase of fluorescence in response to factor Xa, myoglobin, cytochrome C and BSA was negligible (Figure 6). Only trypsin hydrolyzed Compound **1**, but at a slower rate compared to thrombin (7.5-fold more selective for thrombin over trypsin). A commercial assay can detect 1 pM thrombin, by fishing it out of plasma samples using microwells coated with DNA-aptamer. The AMC-based substrate is converted by thrombin to fluorescent product after the enrichment step. The fluorescence assay according to the present invention allowed the detection of thrombin at the concentrations as low as 0.5 pM in water solution (Figure 7), which was achieved without any enrichment step.

### Example 6:

Dabigatran is a commonly used anticoagulant in the clinic. While routine monitoring of dabigatran is not recommended, the determination of its blood level in specific situations (such as bleeding complications, emergency, self-compliance) and/or patient populations (such as the elderly, renal impairment) may increase drug safety. Specific assays for dabigatran have not been established along with drug development and further clinical trials are required to determine the relation of assay results to bleeding or thrombotic complications. In many laboratories only qualitative coagulation-based tests are available, such as prothrombin time (PT) assay or the activated partial thromboplastin time (APTT) assay. Unfortunately these tests often give false-negative results. Other coagulation-based test, such as thrombin clotting time (TCT) detects only minimal dabigatran plasma levels.

Ecarin chromogenic assay (ECA) uses a *p*-nitroanilide substrate and determines accurately therapeutic and supratherapeutic dabigatran levels in plasma.

Described herein is an assay that uses Compound **1** of the present invention for quantification of dabigatran in plasma, and, most importantly, in whole blood, as a key step for the development of a point-of-care test.

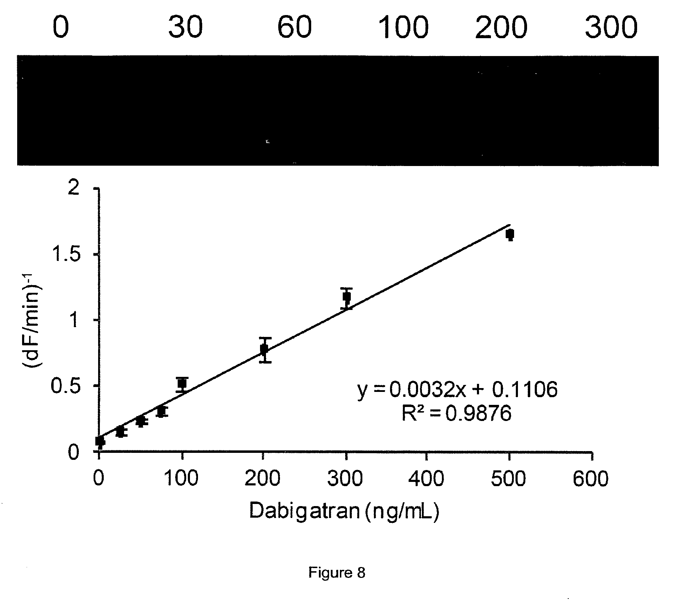

First it was tested how our Compound **1** works in the presence of human plasma. Human plasma was spiked with dabigatran (25-500 ng/mL) and added to a thrombin solution. After incubation for 5 min, the substrate **1** is added and the fluorescence increase at 585 nm is measured over time. A calibration curve could be constructed, which can be used to determine the dabigatran concentration in an unknown sample (Figure 8, lower part). The results of a measurement can even be visualized by naked eye under an UV-lamp (figure 8, upper part).

The next step was to construct a similar calibration curve, but using whole blood instead of plasma.

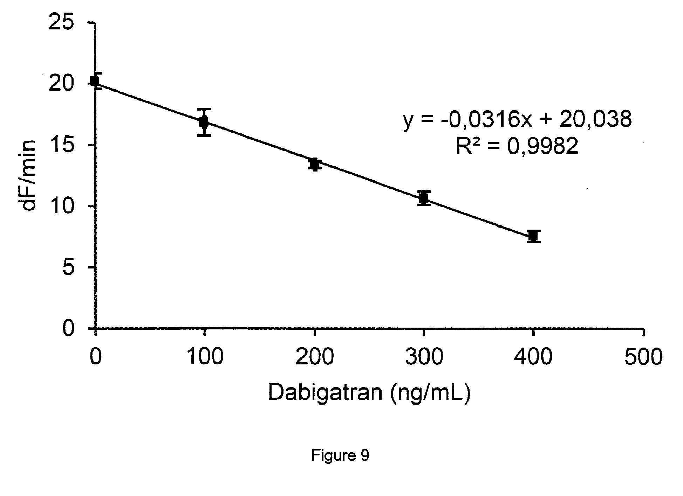

The experimental procedure is similar to the one with plasma. Fresh blood portions (20 µL) were spiked with dabigatran solution (2 µL) and added to thrombin (2 mL) in a single-use fluorescence plastic cuvette. After a short preincubation at room temperature, the fluorogenic substrate was added and the fluorescence change was monitored using a portable fluorescence device (Aquafluor from Turner Designs). Advantageously, it was found that the rate of the enzymatic reaction decreases linearly with the increasing dabigatran concentration (Figure 9).

### Conclusion:

Herein described is the synthesis and the application of a new thrombin substrate **1** based on 3 modules: resorufin fluorophore, self-cleavable PABA linker and recognition tripeptide. Similarly, a new factor Xa substrate **2** was synthesized.

The new substrates are chemically stable toward spontaneous hydrolysis. Fluorogenic Compounds **1** and **2** do not lose their specificity for thrombin and factor Xa correspondingly, if compared to the commercial substrates, due to PABA linker incorporation. Furthermore, **1** and **2** are both chromogenic and fluorogenic substrates: upon reaction with the enzyme it results in more than 300-fold increase in fluorescence; simultaneously a color change from yellow to purple is observed. It could also be shown that compound **1** is 7.5 times more specific for thrombin if compared to trypsin and 400 times more specific for thrombin if compared to factor Xa. Surprisingly, as low as 0.5 pM thrombin in water could be detected using the substrate **1**. This sensitivity is way lower than the aptamer-based methods reported in the literature. Taking advantage of its high selectivity and sensitivity, fluorogenic substrate **1** was applied for quantification of a commonly used anticoagulant dabigatran in the therapeutic range (27-411 ng/mL) in plasma and whole blood. Compound **2** could be used similarly for detection of another important anticoagulant rivaroxaban. The whole blood assay was also adapted for use at the point of care. To our knowledge, this is the first fluorogenic assay which can measure directly the dabigatran concentration without separating the red blood cells.

### Abbreviations:

- AMC:: 7-Amino-4-methylcoumarin
- Boc:: *tert*.-Butyloxycarbonyl
- Bzls:: benzylsulfonyl
- COMU:: 1-Cyano-2-ethoxy-2-oxoethylidenaminooxy)dimethylamino-morpholinocarbenium hexafluorophosphate
- DCC:: *N,N*'-Dicyclohexylcarbodiimide
- DCM:: Dichloromethane
- DIEA:: *N,N*-Diisopropylethylamine
- DME:: Dimethoxyethane
- DMF:: Dimethylformamide
- DMSO:: Dimethyl sulfoxide
- EDTA:: Ethylenediaminetetraacetic acid
- em:: emission
- ex:: excitation
- Et:: ethyl
- FRET:: Forster resonance energy transfer
- Ms:: Methanesulfonyl
- NHS:: *N*-Hydroxysuccinimide
- NP:: nanoparticle
- PABA:: *p*-aminobenzyl alcohol
- Pbf:: 2,2,4,6,7-Pentamethyldihydrobenzofuran-5-sulfonyl
- Ph:: phenyl
- Res:: resorufin
- rt:: room temperature
- TBTU:: *N,N,N',N*'-Tetramethyl-O-(benzotriazol-1-yl)uronium tetrafluoroborate
- TFA:: Trifluoroacetic acid
- THF:: Tetrahydrofuran

## Claims

1. A compound having Formula (I) 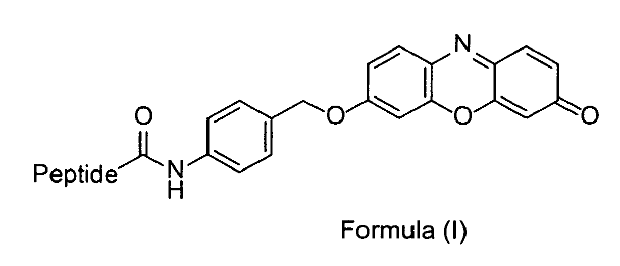 wherein
Peptide is a peptide or peptide derivative, or a salt of said peptide or peptide derivative.

2. The compound according to claim 1, wherein the C-terminal amino acid of said peptide or peptide derivative is Arg or Lys.

3. The compound according to claim 1 or claim 2, wherein the peptide or peptide derivative is selected from the group consisting of thrombin substrates, factor Xa substrates, trypsin substrates, chymotrypsin substrates, factor VIIa substrates, factor IXa substrates, factor XIa substrates, factor XIIa substrates, kallikrein substrates, plasmin substrates, tissue plasminogen activator substrates, activated protein C substrates, tryptase substrates, matriptase substrates, granzyme substrates, elastase substrates, and human complement protease C1r substrates.

4. The compound according to any one of claims 1 to 3, wherein Peptide is the peptide D-Phe-Pro-Arg (Compound **1**) or a salt thereof, or the peptide derivative Benzylsulfonyl-D-Arg-Gly-Arg (Compound **2**) or a salt thereof.

5. A method for the detection of the activity of at least one serine protease in a sample, comprising the steps of contacting said sample with a compound according to any one of claims 1 to 4, and measuring the amount of resorufin released from said compound.

6. The method according to claim 5, wherein the at least one serine protease is a trypsin-like protease.

7. The method according to claim 5 or claim 6, wherein the at least one serine protease is selected from the group consisting of trypsins, chymotrypsins, elastases, thrombin, factor VIIa, factor IXa, factor Xa, factor Xla, factor XIIa, kallikreins, plasmin, tissue plasminogen activator, activated protein C, human complement protease C1 r, tryptases, matriptases, and granzymes.

8. The method according to any one of claims 5 to 7, wherein the at least one serine protease is thrombin or factor Xa.

9. The method according to claim 8, wherein the compound is Compound **1** or a salt thereof, or is Compound **2** or a salt thereof.

10. The method according to any one of claims 5 to 9, wherein the sample (i) is a sample having a high optical density and/or a high autofluorescence, and/or (ii) is in contact with a surface having a high autofluorescence, and/or (iii) contains at least one biological structure that is labeled with at least one chromogenic and/or fluorogenic substance.

11. The method according to any one of claims 5 to 10, wherein the sample is selected from the group consisting of whole blood, serum, plasma, urine, saliva, sputum, semen, lacrimal fluid, cerebrospinal fluid, defecation, cells and tissues.

12. A test strip having a surface on which a compound according to any one of claims 1 to 4 is immobilized.

13. A compound according to any one of claims 1 to 4 for use in the diagnosis of a conditions or disease in a subject that is **characterized by** abnormal levels of at least one serine protease.

14. Use of a compound according to any one of claims 1 to 4 for the detection of the activity of at least one serine protease in a sample.

15. A diagnostic kit, comprising at least one compound according to any one of claims 1 to 4.
